Europäisches Patentamt

**European Patent Office**

Office européen des brevets

(19)

(11) Publication number: **0 144 132**
**A1**

(12) # EUROPEAN PATENT APPLICATION

(21) Application number: **84306922.0**

(22) Date of filing: **10.10.84**

(51) Int. Cl.⁴: **A 61 F 5/00**

(30) Priority: **13.10.83 US 541429**

(43) Date of publication of application:
**12.06.85 Bulletin 85/24**

(84) Designated Contracting States:
**AT BE CH DE FR GB IT LI LU NL SE**

(71) Applicant: Hobbs Medical, Inc.
497 N. Main Street P.O. Box 272
Palmer, MA 01069(US)

(72) Inventor: Hobbs, Eamonn P.
497 N. Main Street
Palmer, MA 01069(US)

(74) Representative: Lynd, Michael Arthur et al,
STANLEY, POPPLEWELL, POOLE 57 Lincoln's Inn Fields
London WC2A 3LS(GB)

(54) **Contraceptive device.**

(57) Contraceptive device, including an inflatable balloon and
a catheter for inserting the balloon in a body passage and
observing the result of inflating the balloon.

Fig. 2

0144132

## CONTRACEPTIVE DEVICE

At the present time, there is only one avenue of permanent contraception available to males, namely, the procedure called "vasectomy". This procedure involves surgery on the vas deferens, the tube that connects the testicle to the uretha and serves as a pathway for the transport of sperm from the testicles to the urethra. In performing a vesectomy procedure, the surgeon cuts each vas deferences in two and ties off each end of the separated tube. At this point, the surgeon has created a break in the pathway of sperm to the urethra and, hence, impregnation of a female by the patent is permanently prevented. The vasectomy procedure is a relatively common one and offers very few complications, but is almost always an irreversible procedure. This irreversibility renders the patient permanently sterile. In other words, the vasectomy suffers from the disability that the procedure involves a certain amount of discomfort and, furthermore, that it is a permanent operation. These and other difficulties experienced with the prior art devices have been obviated in a novel manner by the present invention.

It is, therefore, an outstanding object of the invention to provide a contraceptive device which can be used without the need for surgery..

Another object of this invention is the provision of a procedure for contraception which involves little or no discomfort.

A further object of the present invention is the provision of a male contraceptive procedure which is not irreversible, so that the male is not rendered permanently sterile.

With these and other objects in view, as will be apparent to those ski lled in the art, the invention resides in the combination of parts set forth in the specification and covered by the claims appended hereto.

In general, the invention consists of a contraceptive device that includes an inflatable balloon that is capable of insertion into the vas deferences for the occlusion thereof. The device includes an endoscope which is capable of insertion into the urethra and into the vas deferences. The endoscope includes a catheter or inflation tube and a fibre-optic observation cable. Means is provided for releasably connecting the distal end of the inflation tube to the balloon, while a check valve is associated with the balloon to retain fluid pressure after inflation has been completed.

More specifically, the device includes a substantially rigid elongated hollow occulder body which lies within the balloon and has one end extending from a mouth of the balloon. The said one end of the occluder body is threaded for attachment to the distal end of the catheter. The endoscope includes a tubular jacket within which lies the inflation tube, a bending wire, a fibre-optic light cable, and a fibre-optic observation cable.

The character of the invention, however, may be best understood by reference to one of its structural forms, as illustrated by the accompanying drawings, in which:

Fig. 1 is a sagittal schematic view of a human male showing a contraceptive device embodying the principles of the present invention.

Fig. 2 is a sectional view of the vas deferens of a human male with the device in place,

Fig. 3 is a cross-sectional view of an endoscope forming part of the present invention,

Fig. 4 is a vertical sectional view of a balloon and occluder body forming part of the invention,

Fig. 5 is a transverse sectional view of the balloon and associated equipment taken on the line V-V of Fig. 4, and

Fig. 6 is a detailed view of the occluder body showing a check valve in operation.

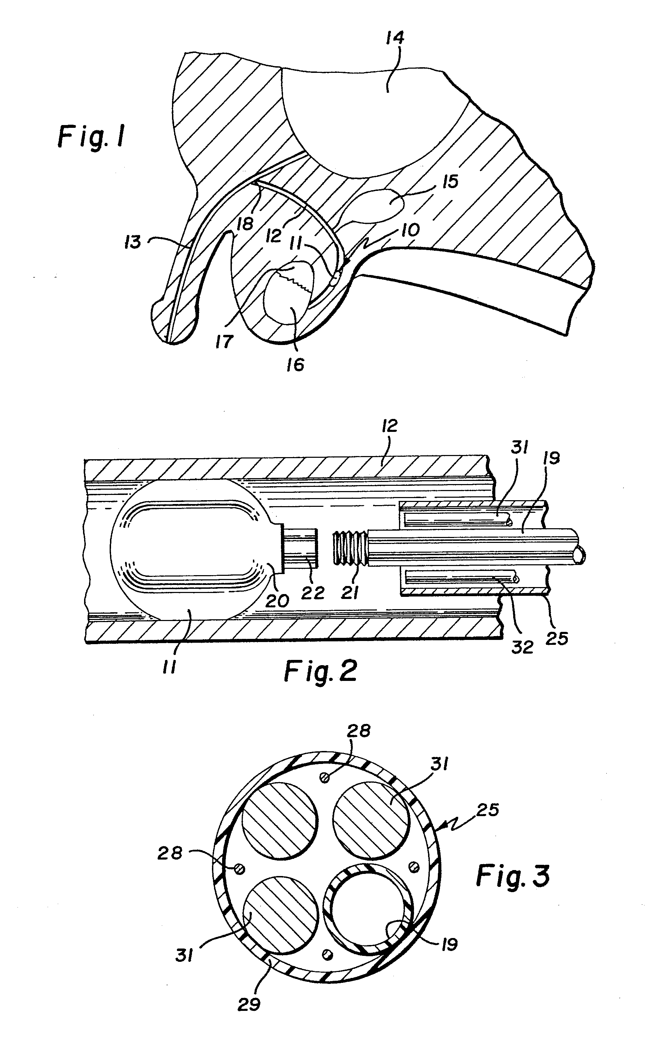

Referring first to Fig. 1, which best shows the general features of the invention, the male contraceptive device, indicated generally by the reference numeral 10, is shown as including a balloon 11 which lies in the vas deferens 12 of a human male. The figure shows the male urethra 13, the urinary bladder 14, and the seminal vesicle 15. Also shown is the testis 16 and the epididymus 17, as well as the ejaculatory duct 18 at the junction between the vas deferens 12 and the urethra 13.

Fig. 2 shows the inflatable balloon 11 lying within the vas deferens 12 at a point between the testis 16 and the seminal vesicle 15. Associated with the balloon is an endoscope 25; the size of the endoscope is selected to be capable of insertion into the urethra 13 and into the vas deferens 12. The endoscope includes a catheter or inflation tube 19 as well as means, consisting of threads 21, for connecting the distal end of the tube to the balloon.

Referring next to Figs. 2 and 3, it can be seen that the endoscope 25 consists of an outer tube or jacket 29 enclosing not only the inflation tube 19, but also bending wires 28, fibre-optic light cables 31 and fibre-optic observation cable 32.

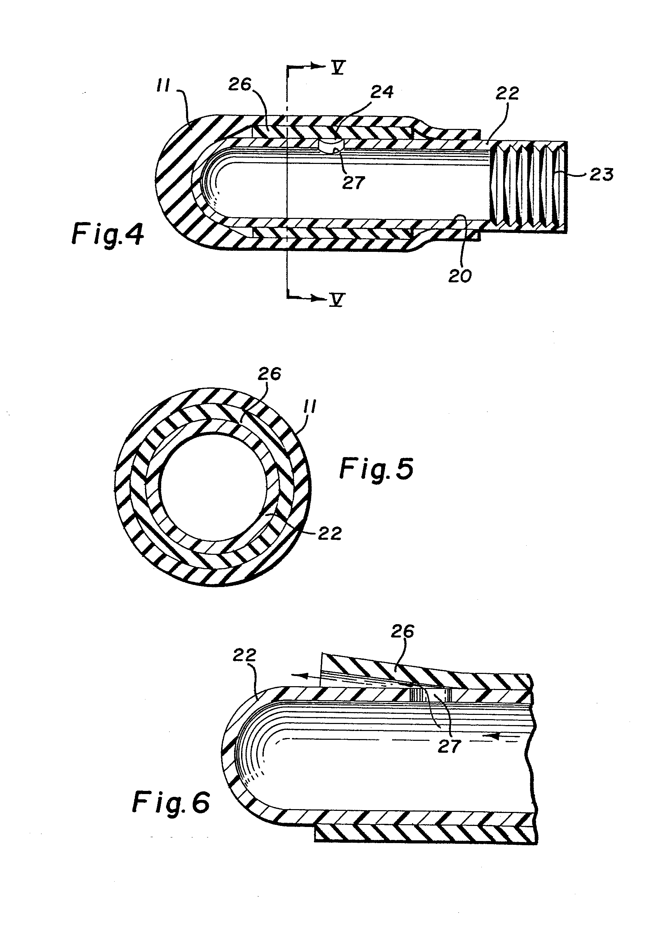

Figs. 4 and 5 show that a substantially-rigid, elongated, hollow occluder body 22 lies within the balloon 11 with one end extending from a mouth 20 of the balloon. The extending end is provided with internal threads 23 for attachment to the distal end of the inflation tubes having the external threads 21. An elastic valve sleeve 26 overlies the occluder body 22 within the balloon. A valve port or aperture 27 is formed in the intermediate portion of the body 22 and is normally closed by the valve sleeve 26. The balloon 11 and the valve sleeve 26 are formed of elastic material, such as rubber, while the occluder body 22 is formed of a rigid plastic, such as polyethylene. In that way, pressure fluid introduced within the hollow occluder body 22 acts to force the valve sleeve away from the port 27 (in the manner shown in Fig. 6), so that the pressure fluid can pass into the balloon 11 to inflate it.

As has been stated before in connection with Fig. 3, the endoscope 25 includes bending wires 28 that permit control of the catheter and balloon, so that they can be passed from the urethra 13 through the ejaculatory duct 18 into the vas deferens 12.

Referring next to Figs. 2 and 3, it can be seen that the endoscope 25 consists of an outer tube or jacket 29 enclosing not only the inflation tube 19, but also bending wires 28, fibre-optic light cables 31 and fibre-optic observation cable 32.

In the preferred embodiment, the tubular jacket 29 of the endoscope 25 has an external diameter of approximately 3.0 millimeters, while the inflation tube 19 has an external diameter of around 1.35 millimeters, each of the light cables 31 has a diameter of around .030 inches and the observation cable has a diameter of around .050 inches.

The operation of the present invention will now be readily understood in view of the above description. The steerable endoscope 25 is introduced into the patient's body through the urethra 13 under direct visual control. The endoscope is advanced in the urethra until the connection of the vas deferens with the urethra (the ejaculatory duct 18) is visually sighted. At this point, the endoscope's distal tip is deflected in such a way as to position itself in the opening of the vas deferens 12. The endoscope is now advanced further along the vas deferens until its distal tip passes the connection between the seminal vesicle 15 and the vas deferens 12. The endoscope is advanced past the seminal vesicle, but still remains in the vas deferens. The endoscope's distal end is now positioned properly for the introduction of the occluding balloon tipped catheter or inflation tube 19. The ballooned tip of the catheter is advanced through the endoscope until it exits the endoscope and enters the visual field of the endoscope. At this time, the inflation media is delivered to the balloon 11 by means of the inflation tube 19, thus inflating the balloon. The amount of inflation media injected into the balloon is measured (along with the balloon's inflation pressure) to ensure that the balloon has reached the proper volume and dimension. The inflated balloon is inspected visually (by means of the light cables 31 and the observation cable 32) to ensure that the balloon has made an adequate seal of the channel of the vas deferens. The catheter or inflating tube 19 is now disconnected from the occluder body 22 and the balloon 11 is withdrawn from the endoscope. The balloon is again visually inspected to ensure that the valve 24 has sealed properly. The endoscope is now withdrawn and the procedure is repeated on the other vas deferens. Both of the vas deferens are now occluded and sperm are prevented from flowing into the urethra.

In the preferred embodiment, the balloon 11 is made from a polymer film which is inert with respect to the environment of the vas deferens. The polymer must have a very low permeability to the gas or liquid which is used to inflate the balloon. Several polymer films can be used to construct the balloon because of their low permeability and chemical resistance; some of them include: silicone, poly(vinylidene chloride), latex rubber, "C-flex" thermoplastic elastomer, polyurethane, and polyethylene.

In the preferred embodiment, that balloon 11 has an internal volume of 0.2 to 1.0 cubic centimeters and has the integral one-way valve 24 to sustain inflation. The balloon is inflated to an internal pressure in the range from 2.0 to 4.0 atmospheres which is adequate pressure to fill the balloon to reach its external volume of 0.2 to 1.0 cubic centimeters and to ensure that the one-way valve 24 has closed off. The balloon can be inflated with a gas or liquid which is radiopaque and non-toxic. An example of a gas that could be used is carbon dioxide and examples of a liquid are a barium water solution and a barium silicone solution.

It can be seen, then, that both occluding balloons 11 when installed serve to prevent transport of sperm to the urethra and make impregnation of a female by the patient impossible. The sperm that builds up behind the occluding balloon are reabsorbed by the body in the same fashion as are the sperm of a recipient of a vasectomy. The occluding balloon can remain implanted indefinitely or can be removed endoscopically by rupturing the balloon with endoscopic forceps and withdrawing it.

To remove the balloon, an endoscope is used to cannulate the vas deferens in the same way as during the installation procedure. When visual contact is made with the balloon, the endoscopic grasping forceps are advanced through the channel up the endoscope and the forceps are used to rupture the balloon and then to grasp it. The endoscope is then withdrawn from the patient's body, while dragging the deflated balloon with it. The patient will now be capable of transporting sperm to the urethra and hence impregnating a female.

It can be seen, therefore, that the procedure for inserting the balloon is relatively painless and involves no surgery. Furthermore, the procedure is 100% effective in rendering the patient temporarily sterile. In addition, this sterility can be terminated in a simple way and very quickly without a hospital stay and the sterility is completely removed.

It is obvious that minor changes may be made in the form and construction of the invention without departing from the material spirit thereof. It is not, however, desired to confine the invention to the exact form herein shown and described, but it is desired to include all such as properly come within the scope claimed.

The invention having been thus described, what is claimed as new and desired to secure by Letters Patent is:

CLAIMS:                    - 12 -

1.    Contraceptive device, comprising:

(a)   an inflatable balloom capable of insertion into a body passage for the occlusion thereof,

(b)   an endoscope capable of insertion into the passage, the endoscope including an inflation tube and a fibre-optic observation cable,

(c)   means for releasably connecting the distal end of the tube to the balloon, and

(d)   a check valve associated with the balloon to retain inflation pressure after inflation has been completed.

2.    Contraceptive device as recited in claim 1, wherein a substantially rigid, elongate, hollow occluder body lies within the balloon and has one end extending from a mouth of the balloon, the said one end being threaded for attachment to the distal end of the inflation tube.

3.    Contraceptive device as recited in claim 2, wherein an elastic valve sleeve overlies the occluder body within the balloon, and wherein a valve part is formed in an intermediate portion of the body and is normally closed by the valve sleeve, pressure fluid within the occluder body acting to form the valve sleeve away from the part, so that the pressure fluid can pass into the balloon for the inflation thereof.

4.    Contraceptive device as recited in any preceding claim, wherein the endoscope includes a bending wire that permits the control of the catheter and balloon in the body passage.

5. Contraceptive device as recited in claim 4, wherein the endoscope includes a tubular jacket within which lies the inflation tube, the bending wire, a fibre-optic light cable, the fibre-optic observation cable.

6. Contraceptive device as recited in claim 5, wherein the tubular jacket has an external diameter of approximately 3.0 millimetres, the inflation tube has an external diameter of around 1.35 millimetres, the light cable has a diameter of approximately 0.30" (076 mm), and the observation cable has a diameter of approximately .050" (1.3mm).

7. Contraceptive device as recited in any one of claims 4 to 6, adapted for use in the male human body, the bending wire permitting control of the catheter and balloon as they pass from the urethra into the vas deferens.

8. A contraceptive device comprising an inflatable balloom capable of insertion into a body passage for the occlusion thereof and a check valve associated with the balloon to retain inflation pressure.

9. A contraceptive device according to claim 8, wherein a substantially rigid elongate hollow occluder body lies within the balloon and has one end extending from a mouth of the balloon, the said one end being threaded for attachment to an inflation tube.

10. A contraceptive device according to claim 9, wherein an elastic valve sleeve overlies the occluder body within the balloon, and wherein a valve part is formed in an intermediate portion of the body and is normally closed by the valve sleeve, pressure fluid within the occluder body acting to form the valve sleeve away from the part, so that the pressure fluid can pass into the balloon for the inflation thereof.

11. A contraceptive device according to claim 9 or 10 adapted for use in the male human body.

12. A contraceptive device substantially as hereinbefore described with reference to Figs. 1, 4, 5 and 6 or with reference to Figs. 1 to 6 of the accompanying drawings.

13. The features hereinbefore disclosed, or their equivalents, in any novel selection.

1/2

0144132

Fig. 1

Fig. 2

Fig. 3

0144132

Fig.4

Fig.5

Fig.6

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| Y | DE-A-2 725 755 (EMSET OY) * Claim 1; page 2, lines 1-5, 11-14 * | 1 | A 61 F 5/00 |
| A | | 8 | |
| Y | DE-A-2 057 181 (HUNTER) * Claims 1, 2, 13-15, 17, 23; figures 12, 13 * | 1 | |
| A | | 2 | |
| Y | US-A-3 866 599 (JOHNSON) * Figure 11 * | 1 | |
| A | US-A-3 402 718 (DOHERTY) * Figure 5 * | 3 | TECHNICAL FIELDS SEARCHED (Int. Cl.4) |
| A | BE-A- 852 513 (CAUDERLIER) | | A 61 B 1/00 A 61 F 5/00 A 61 M 25/00 |
| A | US-A-4 282 875 (SERBINENKO et al.) | | |
| A | GB-A-1 045 202 (PHARMASEAL LAB.) | | |
| | --- -/- | | |

The present search report has been drawn up for all claims

| Place of search BERLIN | Date of completion of the search 11-01-1985 | Examiner KANAL P K |
|---|---|---|

# EUROPEAN SEARCH REPORT

| | DOCUMENTS CONSIDERED TO BE RELEVANT | | Page 2 | |

| Category | Citation of document with indication, where appropriate, of relevant passages | Relevant to claim | CLASSIFICATION OF THE APPLICATION (Int. Cl.4) |
|---|---|---|---|
| A | DE-C- 463 330 (BLUMREICH et al.) | | |
| | --- | | |
| A | DE-U-7 336 834 (R. WOLF GMBH) | | |
| | --- | | |
| A | DE-U-7 535 742 (R. WOLF GMBH) | | |
| | ----- | | |
| | | | TECHNICAL FIELDS SEARCHED (Int. Cl.4) |

The present search report has been drawn up for all claims

| Place of search BERLIN | Date of completion of the search 11-01-1985 | Examiner KANAL P K |
|---|---|---|

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO Form 1503. 03.82